(19) Europäisches Patentamt
European Patent Office
Office européen des brevets

(11) **EP 3 758 761 B1**

(12) **FASCICULE DE BREVET EUROPEEN**

(45) Date de publication et mention de la délivrance du brevet:
**20.04.2022 Bulletin 2022/16**

(21) Numéro de dépôt: **19707014.7**

(22) Date de dépôt: **28.02.2019**

(51) Classification Internationale des Brevets (IPC):
***A61L 9/20*** *(2006.01)*  ***B60H 3/06*** *(2006.01)*

(52) Classification Coopérative des Brevets (CPC):
**A61L 9/205; B60H 3/06;** B60H 2003/0675; B60H 2003/0691

(86) Numéro de dépôt international:
**PCT/EP2019/054978**

(87) Numéro de publication internationale:
**WO 2019/166545 (06.09.2019 Gazette 2019/36)**

(54) **DISPOSITIF DE TRAITEMENT DE L'AIR**

LUFTBEHANDLUNGSVORRICHTUNG

AIR TREATMENT DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.02.2018 FR 1851769**

(43) Date de publication de la demande:
**06.01.2021 Bulletin 2021/01**

(73) Titulaire: **HUTCHINSON**
**75008 Paris (FR)**

(72) Inventeur: **MOUNIER, Jean-Yves**
**45200 Paucourt (FR)**

(74) Mandataire: **Gevers & Orès**
**Immeuble le Palatin 2**
**3 Cours du Triangle**
**CS 80165**
**92939 Paris La Défense Cedex (FR)**

(56) Documents cités:
**EP-A2- 2 765 372  WO-A1-2014/097089**
**FR-A1- 2 838 379  US-A1- 2013 034 470**

**Description**

L'invention concerne un dispositif de traitement de l'air.

Ce dispositif est notamment destiné à être installé dans un moyen de transport (bus, car, avion, hélicoptère, train, véhicule automobile, ...) ou dans un local immobilier (habitation, bureaux, ...). Toutefois, il pourra trouver une application toute particulière dans un véhicule automobile.

Les véhicules automobiles sont aujourd'hui souvent équipés de filtres grossiers, qui sont adaptés pour effectuer de la filtration de polluants dont la taille est typiquement supérieure à 10 microns. Cela concerne des polluants solides, liés soit l'activité humaine (poussières, particules,...) ou d'origine naturelle (pollens, par exemple).

Les véhicules automobiles peuvent aussi être équipés de filtres plus fins, qui sont adaptés pour effectuer de la filtration de polluants dont la taille est comprise entre 0,1 et 10 microns. Les polluants concernés peuvent être des fumées (tabac, pétrole,...), suies ou bactéries.

Les véhicules automobiles peuvent encore être équipés de filtres adaptés pour filtrer des polluants dont la taille est inférieure à 0,1 micron. Les polluants concernés peuvent être des virus et tous les polluants gazeux (CO, N0x et COV). Ainsi, on connaît les filtres à charbon actif.

Un changement régulier du filtre est cependant nécessaire pour s'assurer de la filtration de ces différents polluants. Si le filtre concerné n'est pas changé avant saturation, alors il ne peut plus remplir son rôle de traitement de l'air, voire, dans certains cas, libérer les polluants qui avaient été piégés.

Il existe bien entendu d'autres techniques que celles de l'emploi de tels filtres pour traiter l'air. Ainsi, la photocatalyse est connue pour traiter l'air, en transformant (réaction chimique) les polluants en des composés non polluants. La photocatalyse s'avère notamment intéressante pour traiter les polluants gazeux (CO, NOx et COV) et biologiques (bactéries, virus, allergènes notamment).

FR 2 838 379 décrit un dispositif de traitement de l'air selon le préambule de la revendication 1.

US 2013/034470 décrit un dispositif de traitement de l'air comprenant un photocatalyseur incluant un filtre et une grille de diodes électroluminescentes positionnées sur un cadre à distance du filtre de manière à illuminer le filtre par un rayonnement ultraviolet.

WO 2014/097089 décrit un dispositif d'éclairage pour purifier l'air comprenant une matrice de diodes électroluminescentes positionnée à distance d'un milieu photocatalytique à base de fibres de verre sous la forme d'un feutre servant de filtre. Les diodes sont supportées par un maillage de fils électriques servant de support de sorte que la matrice ne soit pas intégrée dans un support matériel.

EP 2 765 372 décrit un réfrigérateur incluant un filtre métallique à photocatalyse et un module d'éclairage infrarouge comportant une unique grille de diodes électroluminescentes agencées contre ou à distance d'un support poreux pour éclairer le filtre.

Bien que certains des dispositifs existants fassent usage de diodes électroluminescentes, ceux-ci ne permettent pas de réaliser une photocatalyse de manière optimale.

Ainsi, les dispositifs de traitement de l'air basés sur la photocatalyse peuvent être améliorés.

Un objectif de l'invention est ainsi de proposer un dispositif de traitement de l'air mettant en œuvre la photocatalyse, qui est amélioré.

A cet effet, l'invention propose un dispositif de traitement de l'air comprenant :

- un filtre à charbon actif ;
- un photocatalyseur présentant une interface avec le filtre à charbon actif, ledit photocatalyseur comprenant :

    un support poreux présentant un axe longitudinal,
    un revêtement catalytique disposé à la surface du support poreux,
    une pluralité de diodes électroluminescentes agencées, les unes par rapport aux autres, de sorte que l'ensemble du revêtement catalytique puisse être éclairé par lesdites diodes électroluminescentes, les diodes électroluminescentes étant agencées sous la forme d'une pluralité de grilles chacune munie de plusieurs diodes électroluminescentes, chaque grille s'étendant sur l'ensemble d'une section du support poreux, la section étant définie dans un plan perpendiculaire à l'axe longitudinal du support poreux, les différentes grilles étant espacées l'une de l'autre le long de l'axe longitudinal et intégrées dans le support poreux.

Ce dispositif pourra également présenter l'une au moins des caractéristiques suivantes, prises seules ou en combinaison :

- les diodes électroluminescentes sont agencées à une distance non nulle, prise selon ledit axe longitudinal, de l'interface entre le photocatalyseur et le filtre à charbon actif ;
- les grilles sont agencées selon un schéma régulier le long dudit axe longitudinal ;
- les diodes électroluminescentes sont agencées, au sein d'une grille, selon un schéma régulier ;
- les grilles sont identiques ;
- les diodes électroluminescentes sont configurées pour émettre une lumière dans le domaine ultraviolet ;
- les diodes électroluminescentes sont configurées pour émettre une lumière dans le domaine ultraviolet UV-C, en particulier avec une longueur d'onde comprise entre 240nm et 280nm ;

- les diodes électroluminescentes sont configurées pour émettre une lumière avec une longueur d'onde comprise entre 250nm et 260nm ;
- le support poreux est une mousse alvéolaire ;
- le support poreux est réalisé en un matériau choisi parmi un polymère, un métal, du carbone, une céramique ;
- le support poreux est une céramique en carbure de silice, notamment en β-SiC ;
- le revêtement catalytique est choisi parmi le sulfure de cadmium, le sulfure de zinc, l'oxyde de zinc ou le dioxyde de titane, avantageusement en dioxyde de titane.

L'invention sera mieux comprise et d'autres buts, avantages et caractéristiques de celle-ci apparaîtront plus clairement à la lecture de la description qui suit et qui est faite au regard des figures annexées suivantes :

- La figure 1 représente un dispositif de traitement de l'air conforme à l'invention ;
- La figure 2 représente un photocatalyseur connu ;
- La figure 3 représente une grille de diodes électroluminescentes susceptible d'être employée dans le photocatalyseur du dispositif de traitement de l'air représenté sur la figure 1 ;
- La figure 4 représente, en gros plan, un support poreux, en l'occurrence une mousse alvéolaire, susceptible d'être intégré dans le dispositif de traitement de l'air selon l'invention ;
- La figure 5 représente un véhicule automobile comportant un dispositif de traitement de l'air selon l'invention ;
- La figure 6 représente une conception particulière d'un dispositif de traitement de l'air conforme à l'invention ;
- La figure 7 est un schéma représentatif de la géométrie d'un pore d'une mousse alvéolaire employée dans le dispositif de la figure 6.

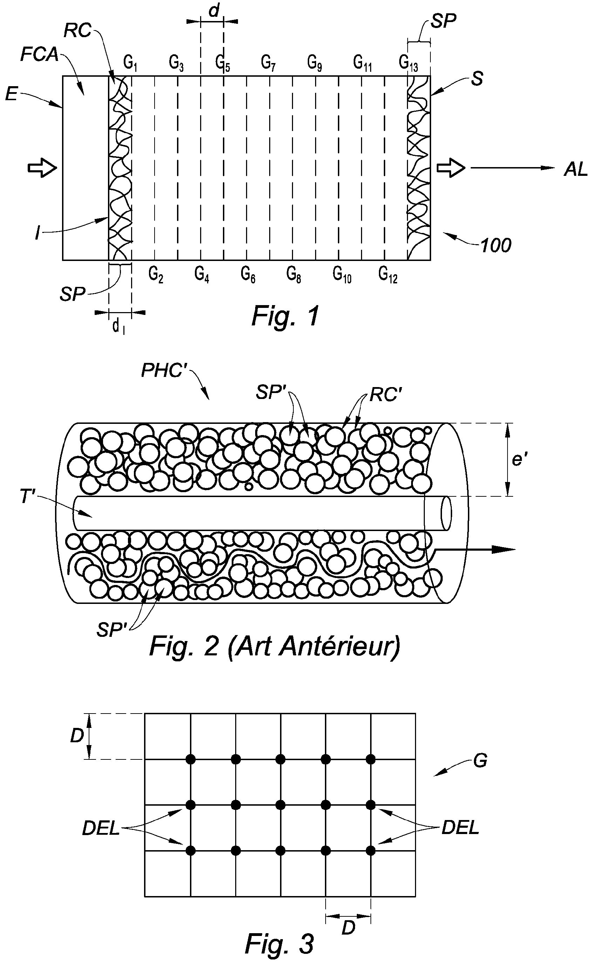

Sur la figure 1, on a représenté un dispositif 100 de traitement de l'air conforme à l'invention. Le dispositif 100 comprend :

- un filtre à charbon actif FCA ;
- un photocatalyseur PHC présentant une interface I avec le filtre à charbon actif, ledit photocatalyseur PHC comprenant :

    un support poreux SP présentant un axe longitudinal AL,
    un revêtement catalytique RC disposé à la surface du support poreux,
    une pluralité de diodes électroluminescentes DEL agencées, les unes par rapport aux autres, de sorte que l'ensemble du revêtement catalytique RC puisse être éclairé par lesdites diodes électroluminescentes.

Sur la figure 1, le support poreux SP et le revêtement catalytique RC ne sont représentés qu'au niveau de l'interface I et de la sortie S du photocatalyseur pour mieux voir le positionnement des DEL, en l'occurrence et à titre d'exemple agencées sous la forme de grilles $G_1$, $G_2$, ..., $G_N$ avec ici N = 13, comportant chacune plusieurs DEL.

Le dispositif 100 de traitement de l'air présente plusieurs intérêts. En fonctionnement, le filtre à charbon actif FCA reçoit des polluants (air pollué : composés organiques volatils (COV) ou autres), les retient puis les laissent passer ou les libère lorsqu'il est à saturation. Le filtre à charbon actif FCA remplit donc tout d'abord son rôle de filtre, mais le fait qu'il ne puisse plus le remplir (saturation) n'est pas une difficulté puisque c'est alors le photocatalyseur PHC qui prend le relais. La présence du filtre à charbon actif en amont du photocatalyseur PHC est en outre mis à profit par le photocatalyseur PHC car la destruction des polluants par le photocatalyseur PHC prend typiquement plusieurs minutes. Pour cette raison, le fait de prévoir un filtre à charbon actif FCA, qui sert de temporisateur, en amont dans le sens de circulation de l'air à traiter permet au photocatalyseur PHC de traiter un maximum de polluants. En l'absence du filtre à charbon actif FCA, il y aurait, pour ce même photocatalyseur, plus de risques que des polluants passent à travers le photocatalyseur sans être traités.

La mise en série du filtre à charbon actif FCA et du photocatalyseur apporte donc un intérêt allant au-delà du simple intérêt procuré par le filtre à charbon actif FCA d'une part ou du simple intérêt procuré par le photocatalyseur d'autre part.

Toutefois, pour que la photocatalyse fonctionne, il convient d'apporter une source de lumière apte à activer celle-ci.

De manière générale, différentes formes de sources lumineuses sont envisageables pour fonctionner avec un catalyseur. La forme la plus employée est un tube (type néon), par exemple émettant dans le domaine ultraviolet (UV), placé au centre du photocatalyseur le long de l'axe longitudinal du support poreux.

C'est ce qui est représenté sur la figure 2 (art antérieur).

Ce type de photocatalyseur PHC' s'avère ne pas être le mieux adapté pour fonctionner avec un filtre à charbon actif situé à proximité immédiate (en amont) du photocatalyseur.

En effet, si le support poreux SP' recouvert d'un revêtement catalytique RC' est nécessaire pour offrir une surface d'échange entre l'air à traiter et le revêtement catalytique RC', ce support poreux SP' fait aussi office de barrière à la transmission de la lumière. Aussi, afin que la lumière puisse être transmise à travers toute l'épaisseur e' (direction radiale) du support poreux SP', le tube T' formant source lumineuse doit émettre une intensité lumineuse relativement importante pour faire en sorte que le revêtement catalytique RC' puisse, en tous points, recevoir une intensité lumineuse apte à permettre la photocatalyse. Plus on s'éloigne du tube T' et moins l'intensité lumineuse reçue par le revêtement catalytique RC' est importante du fait de l'effet d'écran du support poreux SP'.

En conséquence, si un tel photocatalyseur PHC' était installé dans le prolongement d'un filtre à charbon actif, i.e. avec une interface I commune, une partie de la chaleur produite par le tube T' atteindrait le filtre à charbon actif FCA. Il convient en effet de noter que la température de surface d'un tel tube T' (domaine UV) est, en fonctionnement, comprise entre 40° et 90°C en fonction de la puissance émise par le tube. Ainsi et par exemple pour un tube T' fonctionnant dans le domaine UV (250nm), de puissance 35W, il a pu être mesuré une irradiance (éclairement) maximale d'environ 20mW/cm² à 4cm de l'axe central du tube. Le tube présentant un rayon de 0,85cm, on obtient alors une irradiance maximale d'environ 90mW/cm² à la surface du tube. Par ailleurs, à la surface du tube, on a alors une température d'environ 90°C. Une telle température est néfaste dans la mesure où l'apport de chaleur associé aura tendance à libérer plus rapidement les polluants piégés dans le filtre à charbon actif. En effet, quand la température augmente, un filtre à charbon actif désorbe plus, toutes choses égales par ailleurs.

Le gain escompté dans le traitement des polluants par la mise en série d'un filtre à charbon actif et d'un photocatalyseur pourrait en être affecté.

C'est pourquoi, dans le cadre de l'invention, il est non seulement proposé de mettre un photocatalyseur dans le prolongement d'un filtre à charbon actif, i.e. (mise en série) avec une interface commune, mais aussi de prévoir, comme source lumineuse pour la photocatalyse, une pluralité de diodes électroluminescentes DEL et non un tube T' comme dans l'art antérieur.

Une diode électroluminescente présente l'avantage d'émettre une quantité de chaleur bien moindre que celle d'un tube tel que celui qui est illustré sur la figure 2. En conséquence, le risque que la chaleur émise par une diode électroluminescente DEL ait un impact sur le fonctionnement du filtre à charbon FCA actif est, par rapport à un tube du type de celui de la figure 2, bien moindre.

Par ailleurs, dans la mesure où l'irradiance d'une diode électroluminescente est plus faible que celle d'un tube, on comprend qu'il convient de prévoir une pluralité de diodes électroluminescentes. A titre d'exemple non limitatif, on peut prévoir que chaque diode électroluminescente soit fourni par la société LG, sous référence LEUVA66G00HV00 (longueur d'onde = 278nm, puissance optique de 30mW).

De très nombreux agencements de ces diodes électroluminescentes DEL au sein du support poreux SP sont possibles.

En particulier, on peut prévoir que les diodes électroluminescentes DEL soient agencées à une distance $d_l$ non nulle de l'interface I entre le photocatalyseur PHC et le filtre à charbon actif FCA. On minimise encore plus les risques que la chaleur émise par les diodes électroluminescente DEL présente un impact sur le fonctionnement du filtre à charbon actif FCA.

Un agencement particulièrement intéressant des diodes électroluminescentes DEL consiste à former une pluralité de grilles $G_1$, $G_2$, ..., $G_N$, chacune munie de plusieurs diodes électroluminescentes, chaque grille s'étendant sur l'ensemble d'une section du support poreux SP. On notera que la section du support poreux SP est définie dans un plan perpendiculaire à l'axe longitudinal AL du support poreux SP. Les différentes grilles sont alors espacées l'une de l'autre le long de l'axe longitudinal AL du support poreux SP.

Cet agencement sous forme de grilles facilite l'intégration des diodes électroluminescentes dans le photocatalyseur, ainsi que leur répartition.

Avantageusement, les grilles agencées selon un schéma régulier le long de l'axe longitudinal. Autrement dit, la distance d séparant deux grilles qui se succèdent le long de cet axe longitudinal AL est la même.

L'agencement régulier des grilles participe à obtenir une émission de la lumière qui est homogène dans le volume du support poreux.

Avantageusement également, pour chaque grille $G_1$, $G_2$, ..., $G_N$, les diodes électroluminescentes DEL sont agencées selon un schéma régulier. Autrement dit, la distance D (prise dans le plan de la grille, donc perpendiculairement à l'axe longitudinal) séparant deux diodes les plus poches au sein d'une même grille est identique. On a ainsi représenté sur la figure 3, un agencement possible des diodes électroluminescentes au sein d'une même grille selon un agencement régulier.

L'agencement régulier des diodes électroluminescentes au sein d'une grille participe à obtenir d'une émission de la lumière qui est homogène dans le volume du support poreux.

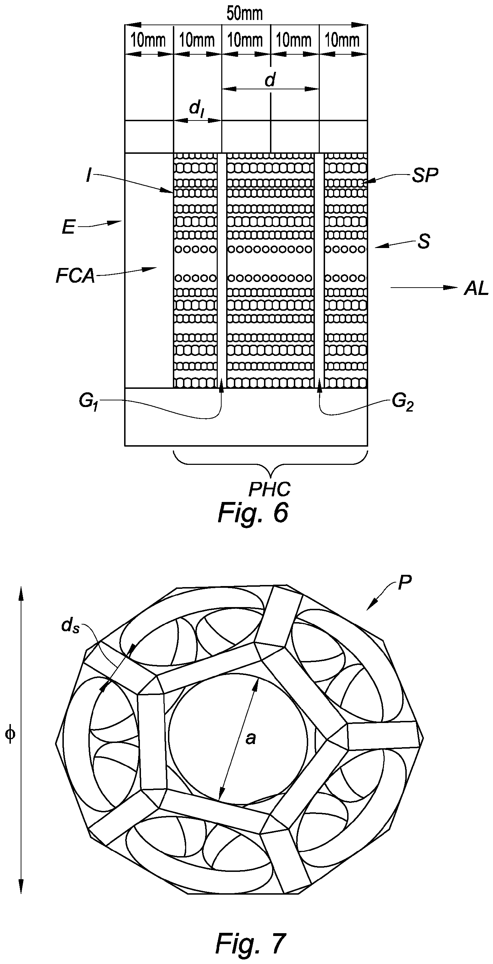

Par ailleurs, si l'on prévoit des grilles dont une seule face est munie de diodes électroluminescentes, alors on peut prévoir que D = d (cas de la figure 1, en combinaison avec la figure 3). Si, en revanche, on prévoit des grilles dont les deux faces sont munies de diodes électroluminescentes, alors on prévoit avantageusement que d = 2D (cas de la figure 6, toujours en combinaison avec la figure 3).

Avantageusement, les grilles qui se succèdent le long de l'axe longitudinal AL du support poreux SP sont identiques. Ceci a pour conséquence l'utilisation de diodes électroluminescentes DEL toutes identiques.

Le fait que les gilles soient identiques participe à obtenir une émission de la lumière qui est homogène dans le volume du support poreux.

Quel que soit l'agencement envisagé pour les diodes électroluminescentes, celles-ci sont alors configurées pour émettre une lumière dans le domaine ultraviolet UV. Le domaine ultraviolet correspond à une longueur d'onde émise par les diodes électroluminescentes DEL typiquement comprise entre 180nm et 400nm.

Il peut être avantageux de fonctionner avec des diodes électroluminescentes émettant une lumière dans le domaine UV-A (longueur d'onde typiquement comprise entre 315nm et 400nm. En effet, le dioxyde de titane est un excellent support catalytique dans ce domaine UV-A et cela est alors particulièrement bien adapté pour le traitement des polluants gazeux.

Il peut également être avantageux de fonctionner avec des diodes électroluminescentes émettant une lumière dans le domaine UV-C (longueur d'onde typiquement comprise entre 180nm et 280nm). Dans ce domaine UV-C, le dioxyde de titane reste un très bon support photocatalytique notamment pour traiter les polluants gazeux et, permet en outre de traiter plus aisément les microorganismes, parmi lesquels les bactéries ou virus. En particulier, il a pu être constaté que pour une longueur d'onde comprise entre 250nm et 260nm, tout particulièrement proche de 254nm, la photocatalyse avait un impact particulier sur le traitement des microorganismes en créant des réactions photochimiques sur leur ADN (acide désoxyribonucléique). L'ADN du microorganisme est alors altéré si bien que le microorganisme perd sa capacité de reproduction.

Le support poreux SP peut présenter différentes géométries.

Cependant, une géométrie particulièrement intéressante est celle d'une mousse alvéolaire. En effet, une mousse alvéolaire présente l'avantage de proposer une surface d'échange spécifique très importante. La surface du revêtement catalytique RC correspondant sensiblement à cette surface spécifique, la mousse alvéolaire est particulièrement efficace pour le traitement d'une quantité importante de polluants. De plus, une mousse alvéolaire, du fait de sa géométrie ouverte, a pour effet de répartir l'air entrant (et plus généralement tout fluide) sur une large étendue (effet de mélange statique), ce qui augmente les chances qu'une particule fluide de l'air puisse atteindre une zone du revêtement catalytique RC, en particulier une zone de ce revêtement catalytique RC non encore déjà prise par un traitement photocatalytique. Enfin, il convient de noter qu'une mousse alvéolaire présente également une perte de charge linéique (en l'occurrence le long de l'axe longitudinal du support catalytique qui correspondra à la direction principale de propagation de l'air pollué à traiter) relativement faible.

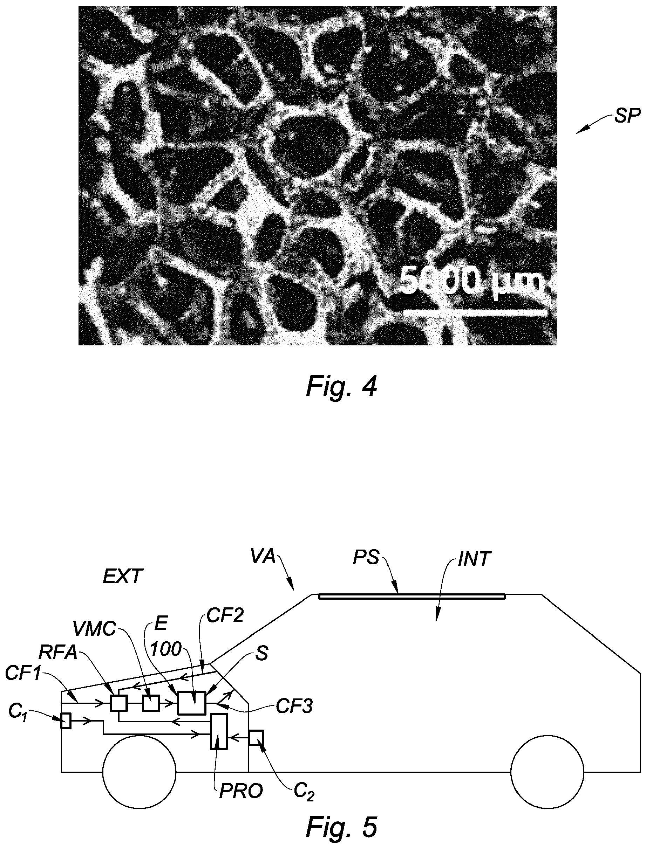

La figure 4 représente une telle mousse alvéolaire susceptible de servir de support poreux.

Par ailleurs, le support poreux SP peut être réalisé avantageusement en matériau polymère, par exemple le polyuréthane, en carbone, par exemple le carbone amorphe, en métal, par exemple le Cuivre ou encore en céramique, par exemple le carbure de silice (SiC), notamment en β-SiC. Le choix particulier du matériau formant le support poreux SP dépend de sa stabilité thermique, chimique et mécanique, des possibilités qu'il offre à fixer certains revêtements photocatalytiques mais également de sa densité, notamment lorsque l'application visée est celle d'un véhicule automobile.

Différents types de revêtements catalytiques peuvent être envisagés, comme par exemple le sulfure de cadmium (CdS), le sulfure de zinc (ZnS), l'oxyde de zinc (ZnO) ou le dioxyde de titane ($TiO_2$). Tous ces revêtements catalytiques peuvent générer une photocatalyse de polluants dans le domaine ultraviolet (UV).

Cependant, le revêtement catalytique RC est avantageusement réalisé en dioxyde de titane ($TiO_2$).

Le dioxyde de titane présente en effet de nombreux avantages pour la photocatalyse :

- un rendement quantique élevé,
- une disposition appropriée de ses bandes de valence et de conduction,
- une très bonne stabilité thermique et chimique.

Pour les applications à la photocatalyse, il est par ailleurs avantageux d'utiliser, pour le dioxyde de titane, les formes allotropiques anatase ou rutile, ou un mélange de ces deux formes.

Une solution particulièrement intéressante est donc de mettre en œuvre une mousse alvéolaire en -SiC, recouverte de $TiO_2$. Des grilles identiques de diodes électroluminescentes émettant dans le domaine ultraviolet (UV) peuvent alors être mises à intervalles réguliers le long de l'axe longitudinal de cette mousse alvéolaire.

Exemple

Pour une application à un véhicule automobile, on pourra par exemple prévoir un dispositif de traitement de l'air avec les caractéristiques suivantes.

Ce dispositif est d'ailleurs représenté sur la figure 6, selon une vue en coupe longitudinale.

Le filtre à charbon actif FCA présente une section (perpendiculaire à l'axe longitudinal du filtre qui se confond avec l'axe global de propagation de l'air à traiter) dont la largeur vaut 25cm et la hauteur 20cm, ainsi qu'une épaisseur (prise selon l'axe longitudinal AL) de 1cm.

Le support poreux SP est une mousse alvéolaire avec une section dont la largeur vaut 25cm et la hauteur 20cm, ainsi qu'une épaisseur d'environ 4cm. Le support poreux est réalisé en -SiC. Le -SiC offre en effet, par rapport à d'autre formes allotropiques du SiC, une bonne stabilité chimique et thermique ainsi que des facilités pour l'accrochage du revêtement catalytique, notamment lorsque ce dernier est du $TiO_2$.

Par ailleurs, les paramètres de base d'un pore P du support poreux SP valent :

- $\Phi = 5400 \pm 700$ microns,

- $a = 2300 \pm 600$ microns,

et

$$- \quad ds = 575 \pm 85 \; microns.$$

où les paramètres géométriques $\Phi$, a et ds sont définis sur la figure 7 (schéma régularisé d'un pore réel).

Le revêtement catalytique RC du support poreux SP est réalisé en $TiO_2$.

Deux grilles $G_1$, $G_2$ de diodes électroluminescentes DEL sont prévues au sein du support poreux SP. Les deux grilles sont identiques. Chaque grille présente des dimensions (largeur, hauteur) identiques à celles de la section du support poreux SP. Au sein d'une grille, la distance D la plus faible séparant deux diodes électroluminescentes vaut 1cm. Chaque grille $G_1$, $G_2$ comporte une même quantité de diodes électroluminescentes de part et d'autre de son plan principal, de sorte à pouvoir assurer un éclairage de part et d'autre de ce plan. Par ailleurs, compte tenu des dimensions d'une grille, chaque grille comporte alors 912 diodes (=24*19*2).

La première grille $G_1$ est située à une distance non nulle, valant en l'occurrence 1cm, de l'interface I entre le filtre à charbon actif FCA et le support poreux SP. La distance inter-grilles d vaut 2cm (d = 2D). En conséquence, la deuxième grille $G_2$ est située à 1cm de la sortie S du photocatalyseur PHC.

Toutes les diodes électroluminescentes DEL sont choisies pour émettre dans le domaine ultraviolet (par exemple UV-C à 254nm), avec une irradiance (prise au niveau de la surface de la diode) d'environ $25mW/cm^2$.

Dans ces conditions, on peut estime que l'ensemble du revêtement catalytique RC reçoit de la lumière, en principe au moins 30% de la lumière émise par l'une des diodes électroluminescentes DEL. Ceci permet de mettre en œuvre l'opération de photocatalyse.

Compte tenu des débits d'air typiquement rencontrés dans un véhicule automobile pour renouveler l'air intérieur dans les normes requises, la conception présentée ci-dessus autorise un temps de séjour suffisant (par exemple 100ms ou plus) à l'air présent dans le photocatalyseur pour être traité.

Fin de l'exemple.

Dans le cadre de l'invention, on vise également un véhicule automobile VA comprenant un dispositif 100 de traitement de l'air tel que décrit précédemment.

On pourra se référer à la figure 5.

Le véhicule automobile VA comprend un premier capteur $C_1$ de pollution agencé, au sein du véhicule, pour mesurer la concentration d'au moins un polluant dans l'air extérieur EXT au véhicule.

Le véhicule automobile comprend aussi un deuxième capteur $C_2$ de pollution agencé, au sein du véhicule, pour mesurer la concentration du même polluant dans l'habitacle INT du véhicule. Le polluant en question peut notamment être un polluant gazeux, par exemple des COV particuliers, les NOx ou le CO.

Le filtre à charbon actif FCA est, au niveau de son entrée E (qui correspond à celle du dispositif 100), en communication fluidique soit avec l'habitacle INT (flèche CF2) du véhicule soit avec l'extérieur EXT (flèche CF1) du véhicule au moyen d'un répartiteur de flux d'air RFA. Le photocatalyseur PHC est, au niveau de sa sortie S (qui correspond à celle du dispositif 100), en communication fluidique (flèche CF3) avec l'habitacle INT du véhicule.

Le véhicule automobile VA comprend également un processeur PRO apte à comparer les concentrations en polluant fournis par les deux capteurs $C_1$, $C_2$ et, en fonction de cette comparaison, à commander le répartiteur de flux d'air RFA de sorte que l'air, provenant de l'extérieur ou de l'habitacle, contenant la concentration en polluant la plus faible soit orienté en direction du dispositif 100 de traitement d'air et plus précisément vers l'entrée E du filtre à charbon actif FCA.

Ceci permet d'envoyer vers le dispositif 100 de traitement de l'air, l'air le moins pollué. On sollicite ainsi moins le dispositif 100 de traitement de l'air, ce qui est avantageux pour sa durée de vie et on minimise les risques que les personnes présentes dans l'habitacle respirent un air pollué.

Dans la mesure où le véhicule est équipé de capteurs de polluants $C_1$, $C_2$, il est également possible d'informer les personnes présentes dans l'habitacle de la nature de la pollution et du niveau de cette pollution. Il suffit à cet égard d'afficher l'information sur un écran du véhicule automobile. Cela peut aider l'automobiliste dans une prise de décision sur sa conduite (changement de parcours, arrêt,...). Il est également possible de proposer une conduite à tenir pour l'automobiliste. Ainsi, si le capteur détecte un taux supérieur aux normes de NOx, il est possible de lui recommander de baisser sa vitesse.

Avantageusement, le véhicule automobile VA présente en outre un panneau solaire PS et une ventilation mécanique contrôlée VMC. La ventilation mécanique contrôlée VMC ainsi que les diodes DEL du dispositif 100 de traitement de l'air sont alors alimentées électriquement par le panneau solaire PS (lien électrique le panneau solaire PS d'une part et la ventilation VMC ainsi que les diodes électroluminescentes DEL d'autre non représentés).

La ventilation mécanique contrôlée VMC est alors agencée, au sein du véhicule, pour véhiculer un flux d'air en direction du dispositif 100 de traitement de l'air.

Ainsi, même à l'arrêt du véhicule automobile (moteur éteint), il est possible de dépolluer l'air, présent soit dans l'habitacle INT du véhicule automobile VA, soit à l'extérieur EXT de ce véhicule VA.

Pour cela, il est avantageux de positionner la ventilation mécanique contrôlée VMC entre le répartiteur de flux d'air RFA et le dispositif 100 de traitement de l'air, car cela permet au choix de traiter l'air de l'habitacle ou l'air extérieur. Dans ce cas, on comprend que le processeur PRO est alimenté électriquement par le panneau solaire PS (lien électrique non représenté), afin que le processeur PRO puisse transmettre une commande au répartiteur de flux RFA. Ainsi, si l'air de l'habitacle est plus pollué que l'air extérieur, alors l'air intérieur sera traité. Dans le cas contraire, c'est l'air extérieur qui sera traité et cela contribue alors, même à l'arrêt du véhicule, à la dépollution de l'air extérieur.

En variante, on peut faire en sorte que le processeur PRO commande, lors de l'arrêt du véhicule, la mise en position du répartiteur de flux d'air RFA sur une entrée de fluide provenant de l'habitacle pour que, lorsque le véhicule est à l'arrêt, seul l'air de l'habitacle soit traité.

## Revendications

**1.** Dispositif (100) de traitement de l'air comprenant :

- un filtre à charbon actif (FCA) ;
- un photocatalyseur (PHC) présentant une interface (I) avec le filtre à charbon actif, ledit photocatalyseur comprenant :

un support poreux (SP) présentant un axe longitudinal (AL),
un revêtement catalytique (RC) disposé à la surface du support poreux,
une pluralité de diodes électroluminescentes (DEL),
ledit dispositif (100) étant **caractérisé en ce que** les diodes électroluminescentes (DEL) sont agencées, les unes par rapport aux autres, de sorte que l'ensemble du revêtement catalytique puisse être éclairé par lesdites diodes électroluminescentes; les diodes électroluminescentes étant agencées sous la forme d'une pluralité de grilles $(G_1, G_2, ...,G_N)$ chacune munie de plusieurs diodes électroluminescentes, chaque grille s'étendant sur l'ensemble d'une section du support poreux (SP), ladite section étant définie dans un plan perpendiculaire audit axe longitudinal (AL) du support poreux (SP), les différentes grilles étant espacées l'une de l'autre le long dudit axe longitudinal et intégrées dans le support poreux (SP).

**2.** Dispositif (100) de traitement de l'air selon la revendication 1, dans lequel les diodes électroluminescentes (DEL) sont agencées à une distance non nulle, prise selon ledit axe longitudinal, de l'interface (I) entre le photocatalyseur et le filtre à charbon actif.

**3.** Dispositif (100) selon la revendication précédente, dans lequel les grilles $(G_1, G_2, ..., G_N)$ sont agencées selon un schéma régulier le long dudit axe longitudinal.

**4.** Dispositif (100) selon l'une des revendications 2 ou 3, dans lequel les diodes électroluminescentes (DEL) sont agencées, au sein d'une grille, selon un schéma régulier.

**5.** Dispositif (100) de traitement de l'air selon l'une des revendications 2 à 4, dans lequel les grilles $(G_1, G_2, ..., G_N)$ sont identiques.

**6.** Dispositif (100) de traitement de l'air selon l'une des revendications précédentes, dans lequel lesdites diodes électroluminescentes (DEL) sont configurées pour émettre une lumière dans le domaine ultraviolet (UV).

**7.** Dispositif (100) de traitement de l'air selon la revendication précédente, dans lequel lesdites diodes électroluminescentes (DEL) sont configurées pour émettre une lumière dans le domaine ultraviolet UV-C, en particulier avec une longueur d'onde comprise entre 240nm et 280nm.

**8.** Dispositif (100) de traitement de l'air selon la revendication précédente, dans lequel lesdites diodes électroluminescentes (DEL) sont configurées pour émettre une lumière avec une longueur d'onde comprise entre 250nm et 260nm.

**9.** Dispositif (100) de traitement de l'air selon l'une des revendications précédentes, dans lequel le support poreux (SP) est une mousse alvéolaire.

**10.** Dispositif selon l'une des revendications précédentes, dans lequel le support poreux est réalisé en un matériau choisi parmi un polymère, un métal, du carbone, une céramique.

**11.** Dispositif (100) de traitement de l'air selon l'une des revendications précédentes, dans lequel le support poreux (SP) est une céramique en carbure de silice (SiC), notamment en β-SiC.

**12.** Dispositif (100) de traitement de l'air selon l'une des revendications précédentes, dans lequel le revêtement catalytique (RC) est choisi parmi le sulfure de cadmium (CdS), le sulfure de zinc (ZnS), l'oxyde de zinc (ZnO) ou le dioxyde de titane $(TiO_2)$, avantageusement en dioxyde de titane $(TiO_2)$.

**13.** Véhicule automobile (VA) comprenant :

- un dispositif (100) de traitement de l'air selon l'une des revendications précédentes dont le filtre à charbon actif (FCA) est, au niveau de son entrée, en communication fluidique soit avec l'habitacle (INT) du véhicule soit avec l'extérieur (EXT) du véhicule au moyen d'un répartiteur de flux d'air (RFA) et dont le photocatalyseur (PHC) est, au niveau de sa sortie, en communication fluidique avec l'habitacle (INT) du véhicule ;
- un premier capteur ($C_1$) de pollution agencé, au sein du véhicule, pour mesurer la concentration d'au moins un polluant dans l'air extérieur au véhicule ;
- un deuxième capteur ($C_2$) de pollution agencé, au sein du véhicule, pour mesurer la concentration du même polluant dans l'habitacle du véhicule ;
- un processeur (PRO) apte à comparer les concentrations en polluants fournis par les deux capteurs ($C_1$, $C_2$) et, en fonction de cette comparaison, à commander le répartiteur de flux d'air de sorte que l'air, provenant de l'extérieur ou de l'habitacle, contenant la concentration en polluant la plus faible soit orienté en direction du dispositif (100) de traitement d'air.

14. Véhicule automobile (VA) selon la revendication précédente comprenant en outre un panneau solaire (PS), par exemple agencé sur le toit du véhicule automobile, et une ventilation mécanique contrôlée (VMC) disposée entre le répartiteur de flux d'air (RFA) et le dispositif (100) de traitement de l'air pour véhiculer un flux d'air en direction dudit dispositif (100) de traitement de l'air, ladite ventilation mécanique contrôlée (VMC) et les diodes électroluminescentes (DEL) étant par ailleurs alimentées électriquement par le panneau solaire.


**Patentansprüche**

1. Vorrichtung (100) zur Behandlung von Luft, umfassend:

   - einen Aktivkohlefilter (FCA);
   - einen Photokatalysator (PHC), der eine Grenzfläche (I) mit dem Aktivkohlefilter aufweist, wobei der Photokatalysator umfasst:

     einen porösen Träger (SP) mit einer Längsachse (AL),
     eine auf der Oberfläche des porösen Trägers angeordnete katalytische Beschichtung (RC),
     eine Vielzahl von elektrolumineszenten Dioden (DEL),
     wobei die Vorrichtung (100) **dadurch gekennzeichnet ist, dass** die elektrolumineszenten Dioden (DEL) in Bezug aufeinander derart angebracht sind, dass die Gesamtheit der katalytischen Beschichtung durch die elektrolumineszenten Dioden beleuchtet werden kann; die elektrolumineszenten Dioden in Form einer Vielzahl von Gittern ($G_1$, $G_2$, ...,$G_N$) angebracht sind, die jeweils mit mehreren elektrolumineszenten Dioden ausgestattet sind, wobei jedes Gitter sich auf der Gesamtheit eines Abschnitts des porösen Trägers (SP) erstreckt, wobei der Abschnitt in einer Ebene senkrecht zu der Längsachse (AL) des porösen Trägers (SP) definiert ist, wobei die unterschiedlichen Gitter entlang der Längsachse voneinander beabstandet und in dem porösen Träger (SP) integriert sind.

2. Vorrichtung (100) zur Behandlung von Luft nach Anspruch 1, wobei die elektrolumineszenten Dioden (DEL) bei einem Abstand, genommen gemäß der Längsachse, von der Grenzfläche (I) zwischen dem Photokatalysator und dem Aktivkohlefilter, von ungleich null angebracht sind.

3. Vorrichtung (100) nach dem vorstehenden Anspruch, wobei die Gitter ($G_1$, $G_2$, ..., $G_N$) gemäß einem regelmäßigen Schema entlang der Längsachse angebracht sind.

4. Vorrichtung (100) nach einem der Ansprüche 2 oder 3, wobei die elektrolumineszenten Dioden (DEL) innerhalb eines Gitters gemäß einem regelmäßigen Schema angebracht sind.

5. Vorrichtung (100) zur Behandlung von Luft nach einem der Ansprüche 2 bis 4, wobei die Gitter ($G_1$, $G_2$, ..., $G_N$) identisch sind.

6. Vorrichtung (100) zur Behandlung von Luft nach einem der vorstehenden Ansprüche, wobei die elektrolumineszenten Dioden (DEL) konfiguriert sind, um ein Licht im ultravioletten Bereich (UV) zu emittieren.

7. Vorrichtung (100) zur Behandlung von Luft nach dem vorstehenden Anspruch, wobei die elektrolumineszenten Dioden (DEL) konfiguriert sind, um ein Licht im ultravioletten UV-C-Bereich zu emittieren, insbesondere mit einer Wellenlänge zwischen 240 nm und 280 nm.

8. Vorrichtung (100) zur Behandlung von Luft nach dem vorstehenden Anspruch, wobei die elektrolumineszenten Dioden (DEL) konfiguriert sind, um ein Licht mit einer Wellenlänge zwischen 250 nm und 260 nm zu emittieren.

9. Vorrichtung (100) zur Behandlung von Luft nach einem der vorstehenden Ansprüche, wobei der poröse Träger (SP) ein alveolarer Schaumstoff ist.

10. Vorrichtung nach einem der vorstehenden Ansprüche, wobei der poröse Träger aus einem Material hergestellt ist, ausgewählt aus einem Polymer, einem Metall, Kohlenstoff, einer Keramik.

**11.** Vorrichtung (100) zur Behandlung von Luft nach einem der vorstehenden Ansprüche, wobei der poröse Träger (SP) eine Keramik aus Siliciumcarbid, besonders aus β-SiC, ist.

**12.** Vorrichtung (100) zur Behandlung von Luft nach einem der vorstehenden Ansprüche, wobei die katalytische Beschichtung (RC) ausgewählt ist aus Cadmiumsulfid (CdS), Zinksulfid (ZnS), Zinkoxid (ZnO) oder Titandioxid ($TiO_2$), vorteilhafterweise Titandioxid ($TiO_2$).

**13.** Kraftfahrzeug (VA), umfassend:

- eine Vorrichtung (100) zur Behandlung von Luft nach einem der vorstehenden Ansprüche, von der der Aktivkohlefilter (FCA) auf Höhe seines Eingangs entweder mit dem Passagierraum (INT) des Fahrzeugs oder mit dem Außenbereich (EXT) des Fahrzeugs mittels eines Luftstromverteilers (RFA) in fluidischer Verbindung vorliegt, und bei der der Photokatalysator (PCT) auf Höhe seines Ausgangs mit dem Passagierraum (INT) des Fahrzeugs in fluidischer Verbindung vorliegt;
- einen ersten Schadstoffbelastungssensor ($C_1$), angebracht innerhalb des Fahrzeugs, zum Messen der Konzentration mindestens eines Schadstoffs in der Außenbereichsluft des Fahrzeugs;
- einen zweiten Schadstoffbelastungssensor ($C_2$), angebracht innerhalb des Fahrzeugs, zum Messen der Konzentration des gleichen Schadstoffs in dem Passagierraum des Fahrzeugs;
- einen Prozessor (PRO), der fähig ist, die von den zwei Sensoren ($C_1$, $C_2$) bereitgestellten Konzentrationen an Schadstoffen zu vergleichen und, abhängig von diesem Vergleich, den Luftstromverteiler so zu befehligen, dass Luft, die aus dem Außenbereich oder dem Passagierraum stammt, die die geringste Konzentration an Schadstoff enthält, in Richtung der Vorrichtung (100) zur Behandlung von Luft gerichtet wird.

**14.** Kraftfahrzeug (VA) nach dem vorstehenden Anspruch, des Weiteren umfassend ein Sonnenpaneel (PA), zum Beispiel auf dem Dach des Kraftfahrzeugs angebracht, und eine gesteuerte mechanische Belüftung (VMC), angeordnet zwischen dem Luftstromverteiler (RFA) und der Vorrichtung (100) zur Behandlung von Luft um einen Luftstrom in Richtung der Vorrichtung (100) zu fördern, wobei die gesteuerte mechanische Belüftung (VMC) und die elektrolumineszenten Dioden (DEL) darüber hinaus durch das Sonnenpaneel elektrisch versorgt werden.

**Claims**

**1.** An air treatment device (100) comprising:

- an activated carbon filter (FCA);
- a photocatalyst (PHC) having an interface (I) with the activated carbon filter, said photocatalyst comprising:

a porous support (SP) having a longitudinal axis (AL),
a catalytic coating (RC) disposed on the surface of the porous support,
a plurality of light-emitting diodes (DEL),
said device (100) being **characterized in that** the light-emitting diodes (DEL) are arranged, relative to one another, such that all of the catalytic coating can be illuminated by said light-emitting diodes; the light-emitting diodes being arranged in the form of a plurality of grids ($G_1$, $G_2$, ... $G_N$) each provided with several light-emitting diodes, each grid extending over all of a section of the porous support (SP), said section being defined in a plane perpendicular to said longitudinal axis (AL) of the porous support (SP), the various grids being spaced apart from each other along said longitudinal axis and integrated into the porous support (SP).

**2.** The air treatment device (100) according to claim 1, wherein the light emitting diodes (DEL) are arranged at a non-zero distance, taken along said longitudinal axis, from the interface (I) between the photocatalyst and the activated carbon filter.

**3.** The device (100) according to the preceding claim, wherein the grids ($G_1$, $G_2$, ..., $G_N$) are arranged in a regular pattern along said longitudinal axis.

**4.** The device (100) according to one of claims 2 or 3, wherein the light emitting diodes (DEL) are arranged, within a grid, in a regular pattern.

**5.** The air treatment device (100) of one of claims 2 to 4, wherein the grids ($G_1$, $G_2$, ..., $G_N$) are identical.

**6.** The air treatment device (100) according to one of the preceding claims, wherein said light emitting diodes (DEL) are configured to emit light in the ultraviolet (UV) range.

**7.** The air treatment device (100) according to the preceding claim, wherein said light emitting diodes (DEL) are configured to emit light in the ultraviolet UV-C range, in particular with a wavelength comprised between 240 nm and 280 nm.

**8.** The air treatment device (100) according to the preceding claim, wherein said light emitting diodes (DEL) are configured to emit light with a wavelength between 250 nm and 260 nm.

**9.** The air treatment device (100) according to one of the preceding claims, wherein the porous support (SP) is a cellular foam.

**10.** The device according to one of the preceding claims, wherein the porous support is made of a material selected from a polymer, a metal, carbon, a ceramic.

**11.** The device (100) for treating air according to one of the preceding claims, wherein the porous support (SP) is a silica carbide (SiC) ceramic, in particular β-SiC.

**12.** The air treatment device (100) according to one of the preceding claims, wherein the catalytic coating (RC) is selected from cadmium sulphide (CdS), zinc sulphide (ZnS), zinc oxide (ZnO) or titanium dioxide ($TiO_2$), advantageously from titanium dioxide ($TiO_2$).

**13.** A motor vehicle (VA) comprising:

- an air treatment device (100) according to one of the preceding claims, whose activated carbon filter (FCA) is, at its inlet, in fluid communication either with the passenger compartment (INT) of the vehicle or with the outside (EXT) of the vehicle by means of an air flow distributor (RFA), and whose photocatalyst (PHC) is, at its outlet, in fluid communication with the passenger compartment (INT) of the vehicle;
- a first pollution sensor ($C_1$) arranged, within the vehicle, to measure the concentration of at least one pollutant in the air outside of the vehicle;
- a second pollution sensor ($C_2$) arranged, within the vehicle, to measure the concentration of the same pollutant in the passenger compartment of the vehicle;
- a processor (PRO) capable of comparing the concentrations of pollutants provided by the two sensors ($C_1$, $C_2$) and, as a function of this comparison, of controlling the air flow distributor so that the air, coming from the outside or from the passenger compartment, containing the lowest concentration of pollutant is directed towards the air treatment device (100).

**14.** The motor vehicle (VA) according to the preceding claim further comprising a solar panel (PS), for example arranged on the roof of the motor vehicle, and a controlled mechanical ventilation (VMC) arranged between the air flow distributor (RFA) and the air treatment device (100) to convey an air flow in the direction of said air treatment device (100), said controlled mechanical ventilation (VMC) and the light-emitting diodes (DEL) being furthermore electrically powered by the solar panel.

*Fig. 1*

*Fig. 2 (Art Antérieur)*

*Fig. 3*

*Fig. 4*

*Fig. 5*

Fig. 6

Fig. 7

**RÉFÉRENCES CITÉES DANS LA DESCRIPTION**

**Documents brevets cités dans la description**

- FR 2838379
- US 2013034470 A
- WO 2014097089 A
- EP 2765372 A